# FASCICULE DE BREVET EUROPEEN

(11) **EP 0 670 840 B1**
(45) Date de publication et mention de la délivrance du brevet: **22.01.1997**
(21) Numéro de dépôt: 94900876.7
(22) Date de dépôt: 25.11.1993
(51) Int. Cl.: C07D 493/08, G21F 9/00, G21F 9/04

(54) **CALIX[4]ARENES-BIS-COURONNES, LEUR PROCEDE DE PREPARATION ET LEUR UTILISATION POUR L'EXTRACTION SELECTIVE DU CESIUM ET DES ACTINIDES**
CALIX-[4]-ARENE-BIS-KRONEN, IHRE HERSTELLUNG UND IHRE ANWENDUNG ZUR SELEKTIVEN EXTRAHIERUNG VON CÄSIUM UND AKTINIDEN
BIS-CROWN CALIX[4]ARENES, METHOD FOR PREPARING SAME AND USE THEREOF FOR SELECTIVELY RECOVERING CESIUM AND ACTINIDES

(30) Priorité: 26.11.1992 FR 9214245
(43) Date de publication de la demande: 13.09.1995
(73) Titulaire: COMMISSARIAT A L'ENERGIE ATOMIQUE, 75752 Paris Cédex 15 (FR)
(72) Inventeur: DOZOL, Jean-François, F-04100 Pierrevert (FR); ASFARI, Zouhair, F-67000 Strasbourg (FR); HILL, Clément, F-84120 Pertuis (FR); VICENS, Jacques, F-67000 Strasbourg (FR)
(74) Mandataire: Des Termes, Monique
(86) Numéro de dépôt international: FR9301161
(87) Numéro de publication internationale: WO9412502

(56) Documents cités:
- US-A- 4 477 377
- JOURNAL OF THE AMERICAN CHEMICAL SOCIETY. vol. 112, no. 19 , 12 Septembre 1990 , GASTON, PA US pages 6979 - 6985 ELEONORA GHIDINI ET AL 'Complexation of alkali metal cations by conformationally rigid,stereoisomeric calix[4]arene crown ethers:A quantitative evaluation of preorganization' cité dans la demande

## Description

La présente invention a pour objet des calix[4]arènes-bis-couronnes, leur procédé de préparation et leur utilisation pour l'extraction sélective du césium et des actinides.

De façon plus précise, elle concerne des calix[4]arènes-bis-couronnes capables d'extraire sélectivement le césium et les actinides présents à l'état de traces dans des solutions acides ayant ou non des concentrations élevées en cations, telles que les effluents aqueux provenant d'installations de retraitement de combustibles nucléaires irradiés ou les solutions de dissolution de combustibles irradiés.

Dans ces effluents, le césium 137 est l'un des produits de fission les plus nocifs du fait de sa longue période (30 ans). Il est donc intéressant de l'éliminer sélectivement des effluents liquides issus des usines de retraitement, en particulier des concentrats d'évaporateurs et des solutions acides possédant ou non une forte salinité due en particulier à la présence de nitrate de sodium.

Etant donné les propriétés chimiques très voisines du sodium et du césium, il est extrêmement difficile d'extraire sélectivement le césium présent dans ces effluents, à une concentration généralement inférieure à 10⁻⁶mol/l, alors que la concentration en sodium est d'environ 4mol/l.

Pour résoudre ce problème, on a envisagé d'extraire le césium au moyen de ligands macrocycliques tels que les para tert-butyl-calixarènes comme il est décrit dans US-A- 4 477 377. Les para tert-butyl-calixarènes utilisés sont le tétramère, l'hexamère et l'octamère, et les meilleurs résultats sont obtenus avec l'hexamère et l'octamère, le tétramère n'ayant pas une très bonne sélectivité pour séparer le césium du potassium. Cette technique d'extraction du césium est intéressante mais elle a pour principal inconvénient de ne s'appliquer qu'au traitement de solutions aqueuses basiques, alors que la plupart des effluents provenant du retraitement sont des solutions acides.

D'autres ligands macrocycliques tels que les éthers-couronnes ont également été utilisés dans ce but comme il est décrit par Dozol et al dans le rapport EUR 13887 FR de la Commission des Communautés Européennes (1992), mais leur sélectivité vis-à-vis du césium reste faible.

La présente invention a précisément pour objet de nouveaux calixarènes qui permettent d'extraire sélectivement le césium à partir de solutions acides et de le séparer du sodium avec une bonne efficacité.

Selon l'invention, les calixarènes sont des calix[4]arènes-bis-couronnes de formule : 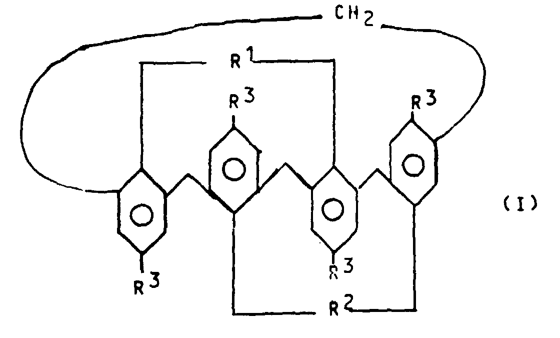 dans laquelle
- R¹ représente X(C₂H₄X)ₘ avec X représentant O, NH et/ou N(CH₃) et m étant égal à 3, 4, 5 ou 6,
- R² représente X(C₂H₄X)ₙ avec X représentant 0, NH et/ou N(CH₃) et n étant égal à 3, 4, 5 ou 6, ou
- R¹ et R² représentent X(C₂H₄X)_{p/2}YX(C₂H₄X)_{p/2} avec X représentant 0, NH et/ou N(CH₃), p étant égal à 2 ou 4 et Y représentant un groupe cyclohexylène, phénylène, naphtylène, pyridylène ou thiophénylène, et
- R³ représente un atome d'hydrogène ou un groupe alkyle en C₁ à C₃.

Dans les chaînons R¹ et/ou R² les X peuvent représenter des atomes d'oxygène qui peuvent être remplacés partiellement ou totalement par NH ou N(CH₃).

Généralement m et n sont identiques et ils sont de préférence égaux à 4 ou à 5.

Ces calixarènes sont très différents des calixarènes utilisés dans US-A- 4 477 377. En effet, dans ce document, on utilisait des calixarènes comportant sur chaque noyau benzénique un groupement phénolique et un groupement tert-butyle, et ces calixarènes ne comportaient qu'un seul macrocycle.

En revanche, dans les calixarènes de l'invention, il y a trois cycles, constitués respectivement par un premier cycle correspondant à l'ensemble des noyaux benzéniques reliés entre eux par les groupes CH₂, un second cycle formé par un pont éther-couronne entre les noyaux benzéniques en positions 1 et 3 et un troisième cycle du type éther-couronne entre les noyaux benzéniques en positions 2 et 4.

Grâce à cette structure particulière, ces calixarènes allient les propriétés complexantes des éthers-couronnes vis-à-vis des alcalins avec les propriétés des calixarènes, c'est-à-dire une importante sélectivité imposée par la structure rigide de la cavité et un caractère lipophile élevé qui rendent la molécule particulièrement intéressante pour l'utilisation dans une membrane liquide supportée.

Les calixarènes de l'invention dans lesquels X représente O peuvent être préparés par un procédé consistant à faire réagir un calix[4]arène de formule : 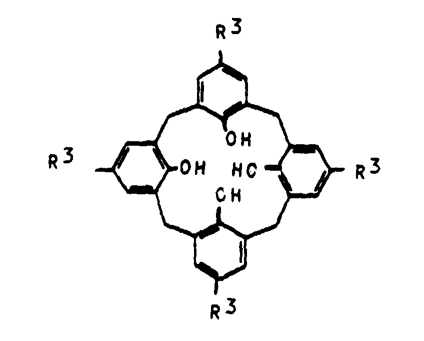 dans laquelle R³ a la signification donnée ci-dessus,
- avec un tétra, penta ou hexa éthylène glycol de formule :

   HOH₂C-(CH₂OCH₂)ₚ-CH₂OH (III)

   dans laquelle p = 2, 3, 4 ou 5, ou
- avec deux glycols différents de formule (III), ou
- avec un glycol de formule

   [HOCH₂(CH₂OCH₂)₂O]₂-Y (IV)

   dans laquelle Y a la signification donnée ci-dessus.

Pour cette réaction, les glycols de formule (III) ou (IV) sont transformés en diparatoluènesulfonates.

Pour effectuer cette réaction, on dissout le calixarène dans un solvant approprié, par exemple dans l'acétonitrile, on y ajoute un sel tel que le carbonate de potassium et on maintient sous agitation pendant au moins 20min à la température ambiante. On ajoute ensuite le tétra, le penta ou l'hexaéthylène glycol de formule (III) ou le glycol de formule (IV) sous la forme de diparatoluènesulfonate et on laisse réagir au reflux pendant une durée suffisante (au moins 8 jours) pour former un pont éther-couronne reliant deux noyaux benzéniques opposés. On ajoute ensuite une nouvelle quantité de tétra, penta ou hexaéthylène glycol sous les formes précédemment décrite-s pour former le second pont éther-couronne. Après réaction, on dissout le mélange réactionnel dans un solvant approprié et on extrait le calixarène, par exemple au moyen d'acide chlorhydrique.

Des calix[4]arènes-bis-couronnes de formule 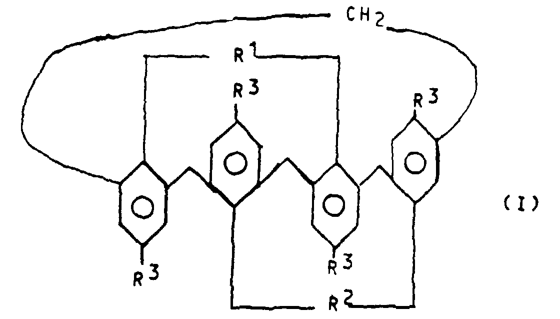 dans laquelle
- R¹ représente X(C₂H₄X)ₘ avec X représentant O, NH et/ou N(CH₃) et m étant égal à 3, 4, 5 ou 6,
- R² représente X(C₂H₄X)ₙ avec X représentant O, NH et/ou N(CH₃) et n étant égal à 3, 4, 5 ou 6, ou
- R¹ et R² représentent X(C₂H₄X)_{p/2}YX(C₂H₄X)_{p/2} avec X représentant O, NH et/ou N(CH₃), p étant égal à 2 ou 4 et Y représentant un groupe cycloalkylène ou arylène, et
- R³ représente un atome d'hydrogène ou un groupe alkyle en C₁ à C₃,
peuvent être utilisés en particulier pour l'extraction sélective du césium présent dans des solutions aqueuses, notamment des solutions acides chargées ou non en sodium telles que les solutions de dissolution et les effluents aqueux provenant d'installations de retraitement de combustibles irradiés.

Lorsque le calix[4]-arène-bis-couronne utilisé comporte des substituants alkyle sur les noyaux benzéniques, il peut être préparé par un procédé analogue à celui décrit ci-dessus en partant du calix|4|arène substitué correspondant.

Un calix[4]arène-bis-couronne substitué de ce type avec R³ représentant le groupe tert-butyle est décrit par exemple par Ghidini et al dans J. Am. Chem. Soc., 1990, 112, p. 6979-6985 mais il est obtenu en tant que produit secondaire, avec un rendement très faible, en utilisant de plus un réactif, tert-butylate de potassium, inflammable et dangereux, et un solvant benzène, inutilisable dans l'industrie.

Pour l'extraction du césium, les solutions aqueuses de départ peuvent être des solutions acides, par exemple des solutions nitriques contenant de 10⁻³ 7mol/l d'acide nitrique.

Pour réaliser cette- extraction, on met en contact la solution aqueuse contenant le césium avec une phase liquide immiscible comprenant le calix[4]arène-bis-couronne, puis on récupère le césium extrait dans la phase liquide immiscible.

Cette récupération peut être effectuée au moyen d'une solution aqueuse par mise en contact de la phase liquide immiscible ayant extrait le césium avec une solution aqueuse de réextraction, constituée par exemple par de l'eau distillée et désionisée.

Pour mettre en oeuvre ce procédé, on peut effectuer la mise en contact de la solution aqueuse avec la phase liquide immiscible dans des installations classiques d'extraction liquide-liquide telles que des mélangeurs-décanteurs, des colonnes d'échange, etc.

On peut aussi effectuer cette mise en contact en disposant la phase liquide immiscible comprenant le calix[4]arène-bis-couronne sous la forme d'une membrane liquide comportant deux surfaces opposées, en faisant circuler la solution aqueuse de départ contenant le césium sur l'une des surfaces de la membrane et en recueillant le césium extrait par la membrane liquide dans une solution aqueuse de réextraction circulant sur la surface opposée de la membrane.

Pour former la phase liquide immiscible, on dissout le calix[4]arène bis-couronne dans un solvant approprié.

A titre d'exemple de solvants utilisables, on peut citer les alkylbenzènes et les nitrophényl alkyl éthers.

De préférence, on utilise comme solvant un éther tel que l'ortho-nitrophénylhexyléther et l'ortho-nitrophényloctyléther.

La concentration en calix[4]arène bis-couronne de la phase liquide immiscible, dépend en particulier du solvant utilisé. On peut utiliser des concentrations allant de 10⁻⁴ à 5.10⁻²mol/l, par exemple une concentration de 10⁻²mol/l.

Les calix[4]arène-bis-couronne de formule (IV) peuvent également être utilisés pour séparer certains actinides de l'américium trivalent. Dans ce cas, on met également en contact la solution aqueuse contenant les actinides avec une phase liquide immiscible contenant le calix|4|arène-bis-couronne pour extraire sélectivement les actinides en conservant l'américium dans la solution aqueuse.

On précise que pour l'extraction du césium comme pour celle des actinides, on peut utiliser les calixarènes sous la forme d'isomères purs ou de mélanges d'isomères. On peut aussi utiliser des mélanges de calixarènes.

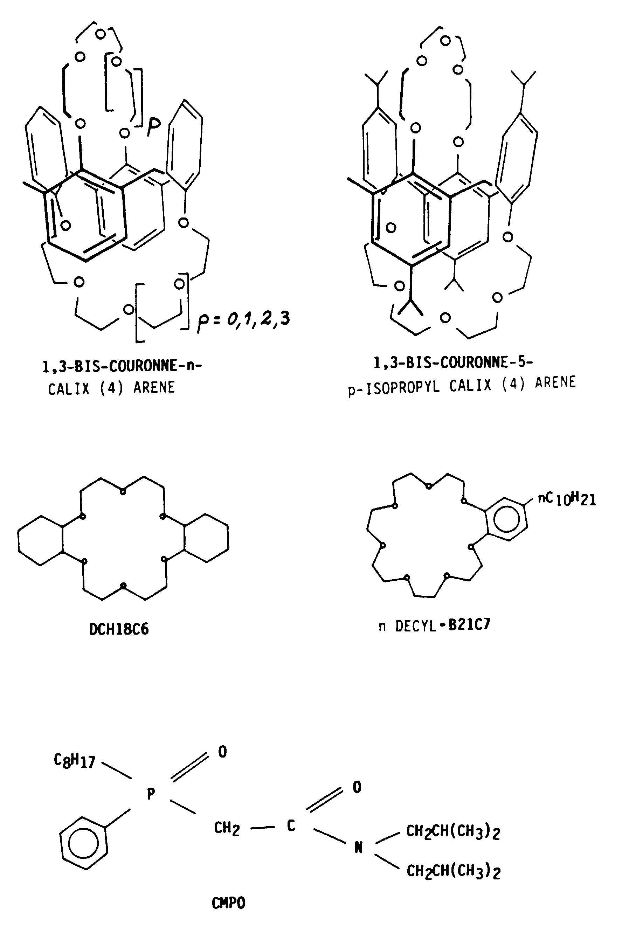

D'autres caractéristiques et avantages de l'invention apparaîtront mieux à la lecture des exemples suivants donnés bien entendu à titre illustratif et non limitatif en référence au dessin annexé qui représente les différents ligands utilisés dans ces exemples.

### Exemple 1 : Préparation du 1,3-bis-couronne-5-calix[4]arène.

Dans un ballon d'un litre muni d'un réfrigérant, on introduit 700ml d'acétonitrile, 6,00g (14,2mmol) de calix[4]arène et 19,62g (142mmol) de carbonate de potassium. On maintient le mélange sous agitation à la température ambiante pendant 20min, puis on y ajoute 7,14g (14,2mmol) de tétraéthylèneglycol-di-p-toluènesulfonate et on porte l'ensemble au reflux sous azote.

Après 8 jours, on ajoute les mêmes quantités de tétraéthylèneglycol di-p-toluènesulfonate, soit 7,12g (14,2mmol) et de carbonate de potassium, soit 19,62g (142mmol) et on laisse le mélange réactionnel au reflux pendant 10 jours.

Après avoir refroidi le mélange réactionnel, on le filtre, on le lave avec du dichlorométhane et on l'évapore au rotavapor. On dissout ensuite le produit dans le dichlorométhane, puis on extrait la solution organique ainsi obtenue avec une solution d'acide chlorhydrique HCl 1N. On concentre le filtrat au rotavapor et on sépare les constituants de l'huile obtenue sur une colonne de silice en utilisant comme éluant un mélange dichlorométhane acétone (85/15 en volume). Le produit recherché est élue en premier et on le précipite dans le méthanol.

On obtient ainsi 1,145g du 1,3-bis-couronne-5-calix[4]arène, ce qui correspond à un rendement de 10%.

Les caractéristiques de ce produit sont les suivantes :
- point de fusion : 117-118°C
- RMN du proton dans CDCl₃ : 7,09ppm (d,8H,J=7,5Hz,Ar-Hméta), 6,89ppm(t,4H,J=7,5Hz,Ar-Hpara), 3,88ppm (s, 8H,Ar-Ch₂-Ar), 3,59-3,06ppm(m,32H,O-CH₂CH₂-O). FAB:m/z=740,3
- analyse élémentaire :
- calculée pour C₄₄H₅₂O₁₀CH₃OH : C=70,02% ; H=7,18%, trouvée : C70,23% ; H=6,92%.

### Exemple 2 : Préparation du complexe césium 1,3-bis-5-couronne-calix[4]arène.

On dissout 53mg (7,16,10⁻²mmol) du 1,3-bis-couronne-5-calix[4]arène obtenu dans l'exemple 1 dans 3ml de chloroforme. Après agitation du mélange, on ajoute un excès de picrate de césium. Après 24h, on filtre le mélange réactionnel et on concentre le filtrat au rotavapor. On obtient ainsi 62mg de complexe à l'état solide, ce qui correspond à un rendement de 99%.

Les caractéristiques du complexe sont les suivantes :
- point de fusion : 73-74°C,
- RMN du proton dans CDCl₃ : 8,87ppm(s, 2H, Ar-H, picrate) 7,3ppm(d, 8H, J=7,28Hz, Ar-H, méta)6,94ppm (m, 4H, Ar-H, para) 3,94ppm-3,24ppm (m, 40H, Ar-CH₂-Ar + O-CH₂-CH₂-O). FAB : m/z(=873,1).

### Exemple 3 : Préparation du 1,3-bis-couronne-6-calix[4]arène.

On maintient sous agitation à la température ambiante, dans un ballon de 250ml, 100ml d'acétonitrile, 850mg (2mmol) de calix[4]arène et 2,764g (20mmol) de carbonate de potassium pendant 20min. On ajoute ensuite 1,14g (2mmol) de pentaéthylèneglycol-di-p-toluènesulfonate et on laisse sous reflux pendant 8 jours. On ajoute alors les mêmes quantités de pentaéthylèneglycol-di-p-toluènesulfonate et de carbonate de potassium et on laisse au reflux pendant 10 jours. On concentre alors le mélange réactionnel au rotavapor, on dissout le solide obtenu dans du dichlorométhane, puis on extrait avec de l'acide chlorhydrique HCl 1N. On sèche la solution organique sur du sulfate de sodium et on la concentre au rotavapor. On précipite l'huile résultante dans l'éther, ce qui donne 662mg du 1,3-bis-couronne-6-calix[4]arène, ce qui correspond à un rendement de 40%.

Les caractéristiques du produit obtenu sont les suivantes :
- point de fusion : 145-146°C,
- RMN du proton dans CDCl₃ : 7,10ppm (d, 8H, J=8Hz, Ar-H méta)6,87ppm (t, 4H, J=8Hz, Ar-H para) 3,87ppm (s, 8H, Ar-CH₂-Ar) 3,70-3,28ppm (m, 40H, O-CH₂-CH₂-O). FAB m/z=828,4.

### Exemple 4 : Préparation du complexe césium-1,3-bis-6-couronne-6-calix|4|arène.

On dissout dans 3ml de chloroforme, 52mg (6,27.10⁻²mmol) du 1,3-bis-couronne-6-calix[4]arène obtenu dans l'exemple 3, puis on ajoute un excès de picrate de césium et on maintient l'ensemble sous agitation pendant 24h. On filtre alors la solution et on concentre le filtrat au rotavapor, ce qui donne 74mg de complexe à l'état solide, soit un rendement de 99%.

Les caractéristiques du complexe sont les suivantes :
- point de fusion : 79-80°C
- RMN du proton dans CDCl₃ : 8,87 ppm (s, 2H, Ar-H, picrate) 7,27-7,22ppm (m, 8H, Ar-H, meta) 7,06-6,98ppm (m, 4H, Ar-H, para)3,87-3,58ppm (m, 48H, ArCH₂-Ar+O-CH₂-CH₂-O). FAB m/z=961,3.

### Exemple 5 : Préparation du 1,3-bis-couronne-7-calix[4]arène.

On suit le même mode opératoire que dans l'exemple 1 pour préparer le 1,3-bis-couronne-7-calix[4]arène en utilisant au lieu de tétraéthylèneglycol-di-p-toluènesulfonate, de l'hexaéthylèneglycol-di-p-toluènesulfonate. On obtient le 1,3-bis-couronne-7-calix[4]arène, avec un rendement de 18%

Les caractéristiques de ce produit sont les suivantes :
- point de fusion : 120-121°C,
- RMN du proton dans CDCl₃ : 7,14ppm (d, 8H, J =7,5Hz, Ar, H, meta) 6,80ppm (t, 4H, J = 7,5 Hz Ar - H, para) 3,72 - 3,64 (m, 56H, O - CH₂-CH₂-O+Ar-CH₂-Ar)-F.A.B m/z=916,4.
Analyse élémentaire : calculée pour C₅₂H₆₈O₁₄
C:68,10% H:7,47% - Trouvé : C:68,30% H:7,27%.

### Exemple 6 : Préparations des 1,3bis(benzo 1,4 ou 1,2 bis éthylène glycol)calix[4]arènes.

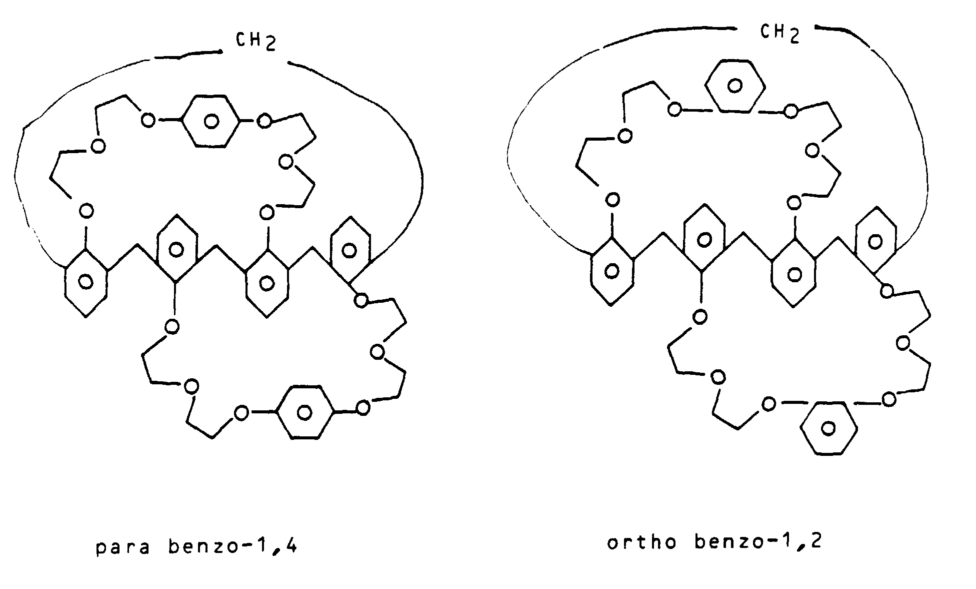

a) Pour le para : on suit le même mode opératoire que dans l'exemple 3 pour préparer le 1,3-bis-(benzo-1,4-bis éthylèneglycol)-calix[4]arène en utilisant le 1,4-bis(toluène para éthoxyéthoxysulfonate)-benzène au lieu du pentaéthylèneglycol di-paratoluéne sulfonate.
   On obtient le composé "benzo-1,4" avec un rendement de 54%.
   - point de fusion 81-82°C.
   - RMN (CDCl₃) 7-07 (d, 8H, J = 7,5 Hz, ArH meta)
      7,03 (s, 8H, ArH, benzène)
      6,83 (t, 4H, J=7,5Hz, ArH para)
      3,82 - 3,67 (m, 40H, -OCH₂CH₂O- + Ar CH₂ Ar)
   - FAB m/z = 924,4
b) Pour le ortho : On suit le même mode opératoire que dans l'exemple 3 pour préparer le 1,3-bis(benzo-1,2-bis-éthylèneglycol)-calix[4]arène en utilisant le 1,2-bis (toluène para éthoxy éthoxy sulfonate)-benzène au lieu du pentaéthylèneglycol di-paratoluène sulfonate. On obtient le composé "benzo-1,2" avec un rendement de 62%.
   - point de fusion 188-189°C
   - RMN (CDCl₃)
   - RMN du proton dans CDCl₃ 7,07ppm (d, 8H, j=7,5 Hz, Ar-H, meta) 6,99ppm (s, 8H, Ar-H catéchol) 6,80 ppm (t, 4H, J=7,4Hz, Ar-H, para) 3,72-3,64 ppm (m, 40 H, O-CH₂-CH₂-O + Ar-CH₂-Ar). FAB m/z=925,6. Analyse élémentaire : calculée pour C₅₆H₆₀O₁₂:C 72,70%, H 6,54%;C 72,42%, H 6,41%.

### Exemples 7 à 14 : Extraction du césium.

Dans ces exemples, on extrait le césium présent dans une solution aqueuse ayant une concentration en acide nitrique de 0,97 mol/l, contenant 5.10⁻⁴mol/l de CsNO₃.

Dans ce but, on met un volume de solution aqueuse en contact avec un volume de liquide organique constitué par de l'ortho-nitrophénylhexyl éther contenant 10⁻²mol/l d'extractant organique. Lorsque l'équilibre est atteint, on mesure par spectrométrie gamma, la teneur en césium du liquide organique. On détermine ensuite le pourcentage de césium extrait et le coefficient de distribution D_{Cs} qui correspond au rapport de la concentration en césium dans le liquide organique à la concentration en césium de la solution aqueuse à l'équilibre.

Dans ces exemples, on utilise comme extractant organique les ligands macrocycliques du tableau 1 dont les formules sont données sur la figure annexée. Les résultats obtenus sont également donnés dans le tableau 1.

Dans ces exemples, les exemples 7 à 12 concernent l'utilisation des calix[4]arènes bis-couronnes de l'invention, et les exemples 13 et 14 donnés à titre comparatif illustrent l'emploi d'éthers couronnes comme extractants.

Les résultats du tableau 1 montrent que le calixarène utilisé dans l'exemple 10 (calixarène de l'exemple 6b) permet d'obtenir un très bon taux d'extraction.

Par ailleurs, le césium extrait dans cet exemple peut être récupéré avec un taux de 77% par mise en contact de la phase organique avec de l'eau déminéralisée, le pH à l'équilibre étant de 2,7.

### Exemples 15 à 20 : Extraction du strontium.

On part d'une solution aqueuse ayant une concentration en acide nitrique de 1,2mol/l contenant 5.10⁻⁴mol/l de nitrate de strontium, et on la soumet à une extraction dans les mêmes conditions que celles des exemples 7 à 14 en utilisant une phase liquide organique constituée par de l'ortho-nitrophényl hexyl éther contenant 10⁻²mol/l d'extractant macrocyclique.

On mesure ensuite la teneur en strontium de la phase organique par spectrométrie gamma et on détermine le pourcentage de strontium extrait et le coefficient de distribution D_{Sr}.

Les extractants utilisés et les résultats obtenus sont donnés dans le tableau 2.

### Exemples 21 à 27 : Extraction du sodium.

Dans ces exemples, on part d'une solution aqueuse ayant une concentration en acide nitrique de 0,97mol/l contenant 5.10⁻⁴mol/l de nitrate de sodium, et on la soumet à une extraction dans les mêmes conditions que celles des exemples 7 à 14 en utilisant un extractant macrocyclique à une concentration de 10⁻²mol/l dans de l'ortho-nitrophényl hexyl éther. On mesure comme précédemment la teneur en sodium de la solution organique par spectrométrie gamma et on évalue le pourcentage de sodium extrait et le coefficient de distribution D_{Na}.

Les extractants et les résultats obtenus sont donnés dans le tableau 3.

Les résultats donnés dans les tableaux 1 à 3 montrent que les calix[4]arènes-bis-couronnes utilisés dans l'invention extraient le césium avec de bons rendements et le séparent du sodium et du strontium présents également dans les effluents issus des usines de retraitement.

A partir des résultats des tableaux 1 à 3, on peut calculer les sélectivités des différents extractants vis-à-vis de ces cations en milieu nitrique.

On trouve ainsi pour la sélectivité α(Cs/Na) = D_{Cs} / D_{Na} relative au césium par rapport au sodium les valeurs suivantes :18000 pour le 1,3-bis(benzo 1,2-bis-éthylèneglycol)calix[4]arène, 1500 pour le 1,3-bis-couronne-6-calix[4]arène, 1250 pour le 1,3-bis-couronne-7-calix[4]arène, 200 pour le 1,3-bis-couronne-5-calix[4]arène, 126 pour le 1,3-bis-couronne-5-O-isopropyl calix[4]arène,
alors que cette sélectivité est de 250 pour l'éther couronne B21C7.

Pour la sélectivité αCs/Sr relative au césium par rapport au strontium, on trouve les valeurs suivantes :
>3000 pour le 1,3-bis-couronne-7-calix[4]arène et
4500 pour le calix[4]arène-bis-couronne-5,
alors que cette sélectivité n'est que de 7,6.10⁻² pour l'éther couronne DCH18C6.

C'est pourquoi les calixarènes de l'invention permettent d'obtenir des transports sélectifs de césium 137, à l'état de traces dans une matrice de nitrate de sodium 4M et d'acide nitrique 1M contenant d'autres produits de fission tels que du Sr 85 également à l'état de traces.

Les résultats des tableaux 1 à 3 montrent par ailleurs que les 1,3-bis-couronne-6-calix[4]arènes sont les meilleurs extractants du césium dans la famille des calix[4]arène-bis-couronne. Ce pic de sélectivité pourrait s'expliquer par une bonne adéquation entre la taille des cavités disponibles et la taille du cation extrait. Pour les 1,3 bis-couronne-6-calix[4]arènes, la présence d'un groupement benzo-1,2 améliore encore cette sélectivité vis-à-vis du césium.

### Exemples 28 à 33 : Extraction des actinides.

Dans ces exemples, on part d'une solution aqueuse contenant 1mol/l d'acide nitrique et 4mol/l de nitrate de sodium, contenant des traces de Np 237, de Pu 239 et de Am 241 et on extrait les actinides en utilisant une phase organique liquide constituée par un extractant macrocyclique à une concentration de 10⁻²mol/l dans l'ortho-nitrophényl octyl éther ou l'orthonitrophénylhexyléther, ou un extractant connu (CMPO) à une concentration de 10⁻²mol/l dans l'ortho-nitrophényl hexyl éther.

On mesure ensuite les quantités de ²³⁷Np, ²³⁹Pu et ²⁴¹Am présentes dans la solution organique et on détermine les coefficients de distribution du neptunium, du plutonium et de l'américium entre les deux phases en contact.

Les extractants utilisés et les résultats obtenus sont donnés dans le tableau 4.

Au vu de ces résultats, on remarque que les calix[4]arène-bis-couronne de l'invention sont de moins bons extractants que le CMPO mais qu'ils sont plus sélectifs puisqu'ils n'extraient pas l'américium trivalent.

Ainsi, on peut effectuer avec le 1,3-bis-couronne-6-calix[4]arène des expériences de transport en disposant la phase liquide organique sous la forme d'une membrane liquide séparant la solution aqueuse de départ d'une solution de réextraction constituée par de l'eau déminéralisée, et décontaminer plus de 95% de Pu 239 et plus de 50% de Np 237 en moins de 48h, sans transporter d'américium.

Les calix[4]arènes-bis-couronnes de l'invention peuvent aussi être utilisés en analyse, par exemple pour déterminer l'activité des différents isotopes du césium, en particulier du Cs-135. Ils peuvent être encore utilisés en détoxication.

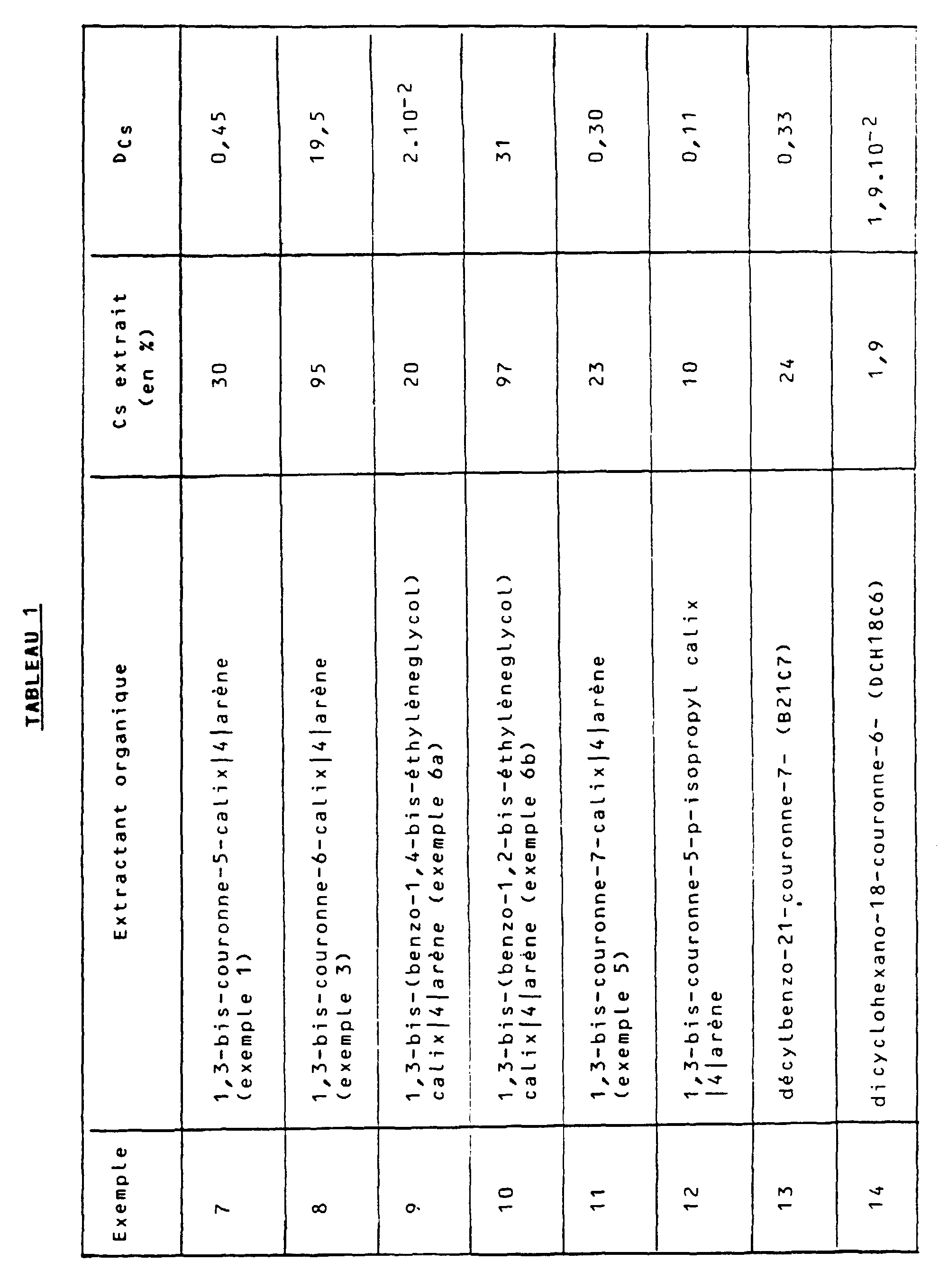

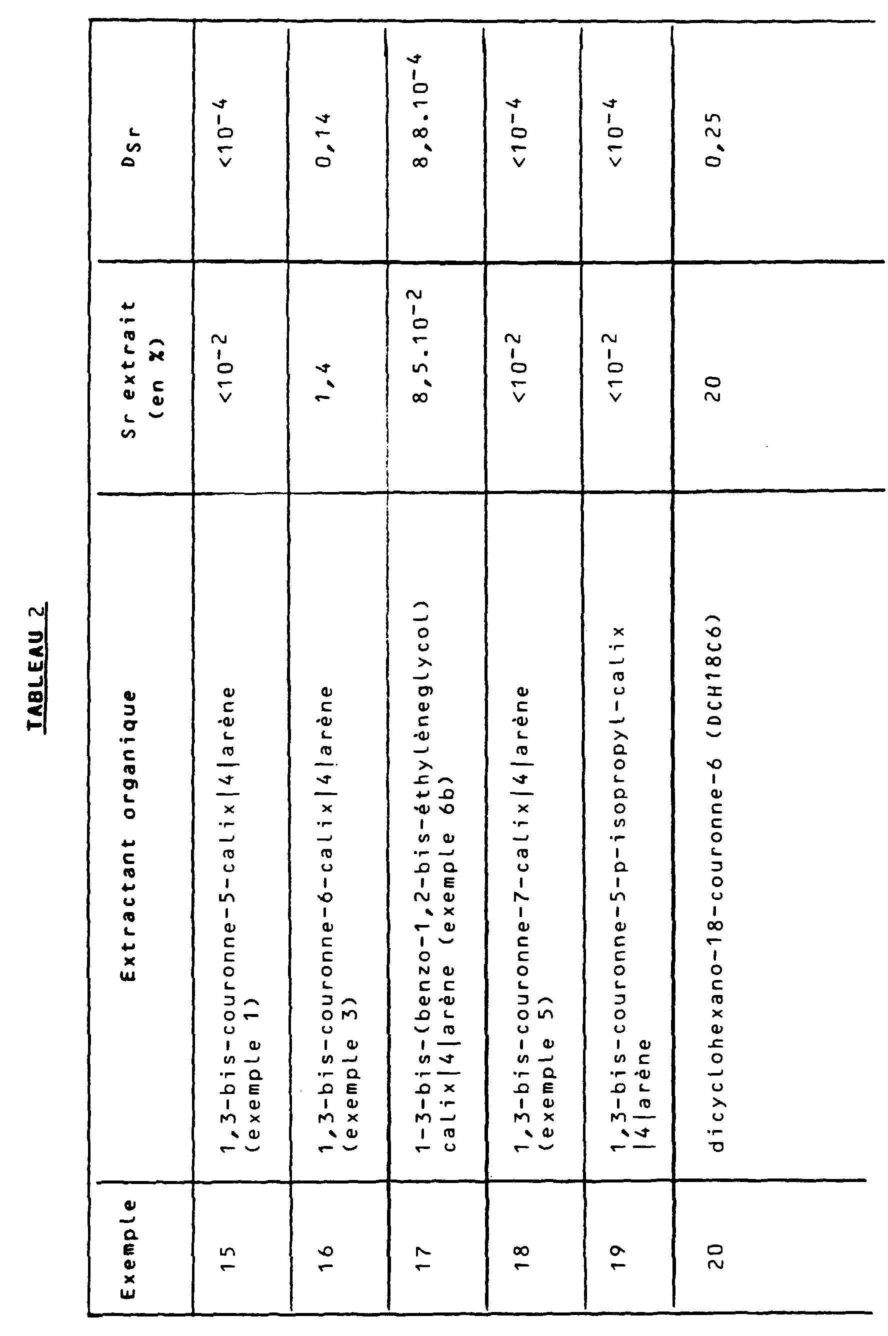

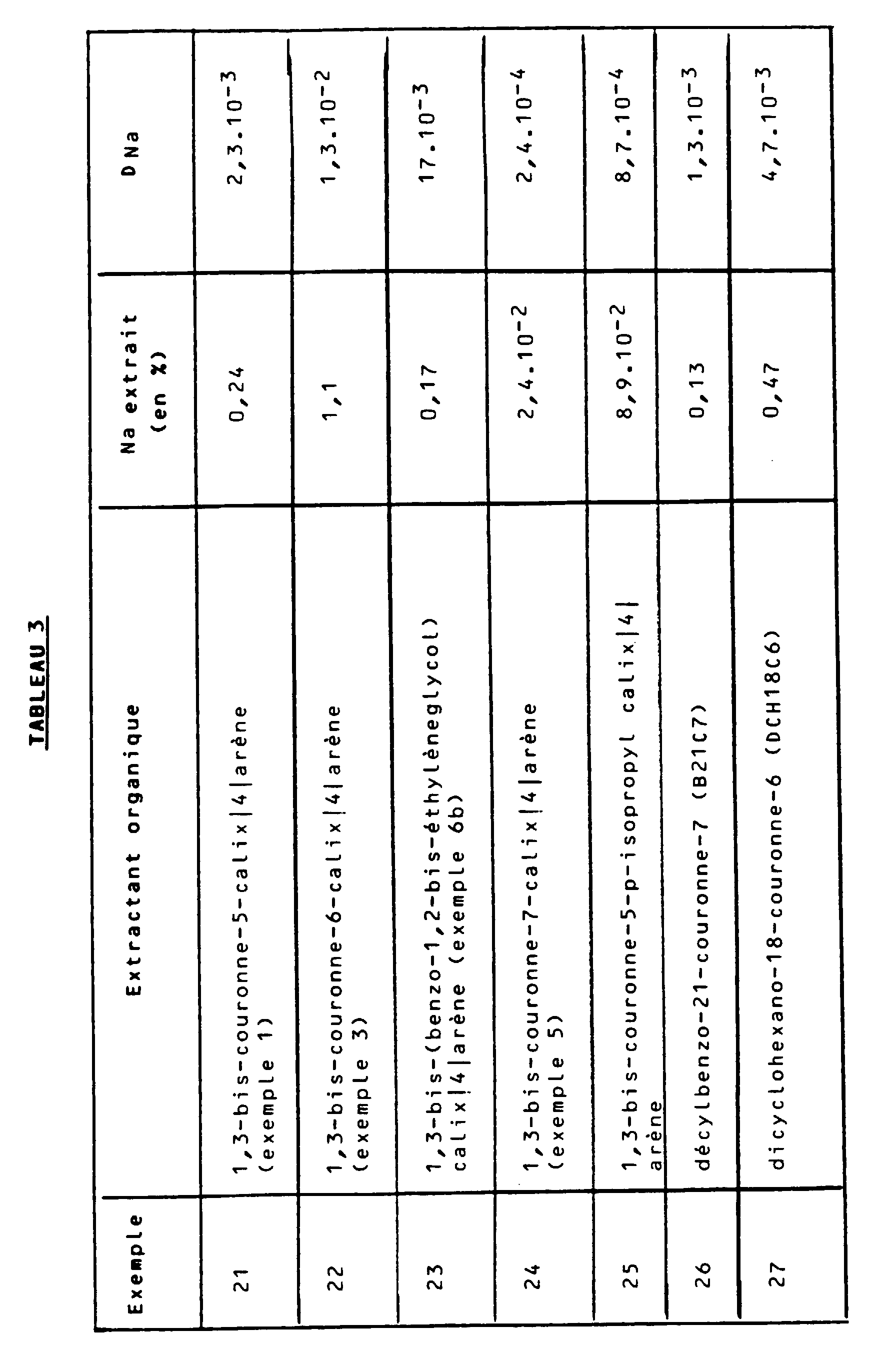

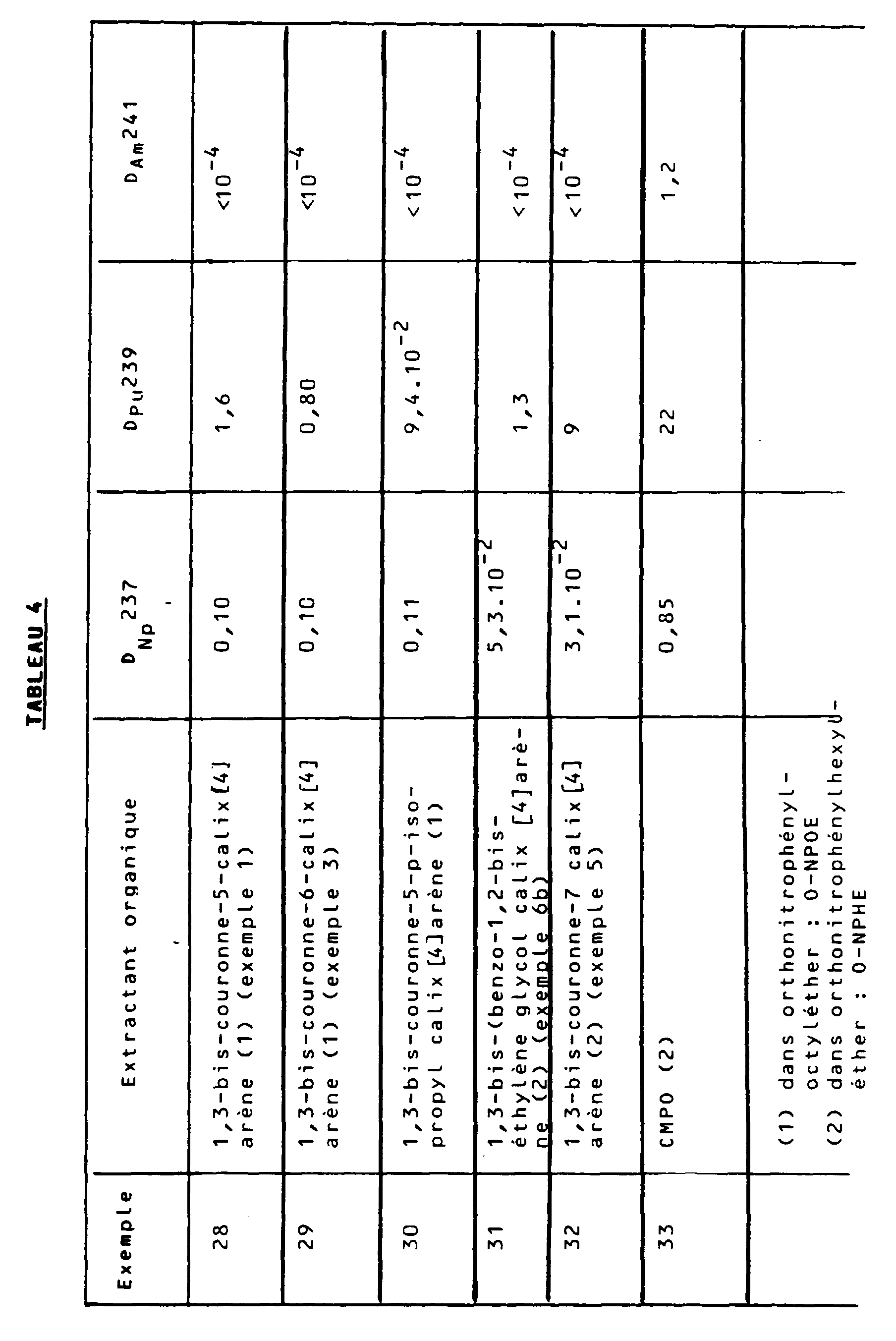

## Revendications

1. Calix[4]arène-bis-couronne de formule : 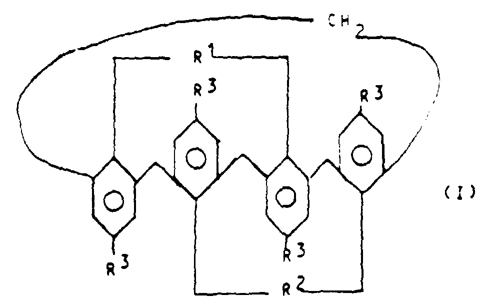 dans laquelle
- R¹ représente X(C₂H₄X)ₘ avec X représentant O, NH et/ou N(CH₃) et m étant égal à 3, 4, 5 ou 6,
- R² représente X(C₂H₄X)ₙ avec X représentant O, NH et/ou N(CH₃) et n étant égal à 3, 4, 5 ou 6, ou
- R¹ et R² représentent X(C₂H₄X)_{p/2}YX(C₂H₄X)_{p/2} avec X représentant O, NH et/ou N(CH₃), p étant égal à 2 ou 4 et Y représentant un groupe cyclohexylène, phénylène, naphtylène, pyridylène ou thiophénylène, et
- R³ représente un atome d'hydrogène ou un groupe alkyle en C₁ à C₃.

2. Calix[4]arène-bis-couronne selon la revendication 1, caractérisé en ce que R¹ et R² représentent O(C₂H₄O)₄, et R³ représente un atome d'hydrogène.

3. Calix[4]arène-bis-couronne selon la revendication 1, caractérisé en ce que R¹ et R² représentent O(C₂H₄O)₅ et R³ représente un atome d'hydrogène.

4. Calix[4]arène-bis-couronne selon la revendication 1, caractérisé en ce que R¹ et R² représentent 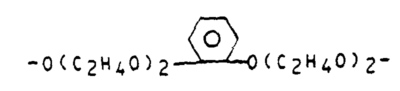 et R³ représente un atome d'hydrogène.

5. Procédé de préparation d'un calix[4]arène-bis-couronne pour lequel X représente O selon l'une quelconque des revendications 1 à 4, caractérisé en ce qu'il consiste à faire réagir un calix[4]arène de formule : 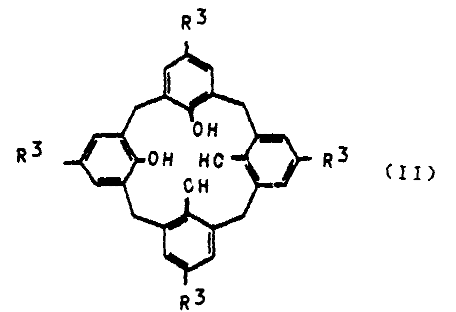 dans laquelle R³ a la signification donnée dans la revendication 1,
- avec un tétra, penta ou hexa éthylène glycol de formule :
HOH₂C-(CH₂OCH₂)ₚ-CH₂OH (III)
dans laquelle p = 2, 3, 4 ou 5, ou
- avec deux glycols différents de formule (III), ou
- avec un glycol de formule
[HOCH₂(CH₂OCH₂)₂O]₂-Y (IV)
dans laquelle Y a la signification donnée dans la revendication 1.

6. Procédé pour séparer le césium d'une solution aqueuse, caractérisé en ce qu'il consiste à mettre en contact cette solution aqueuse avec une phase liquide immiscible comprenant un calix[4]arène-bis-couronne de formule : 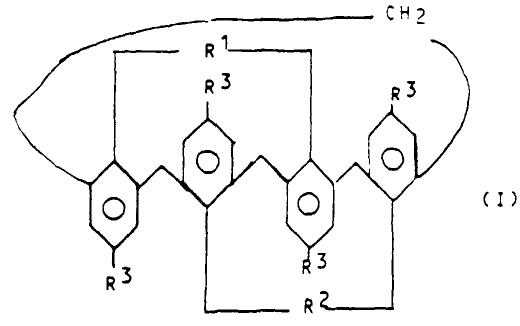 dans laquelle
- R¹ représente X(C₂H₄X)ₘ avec X représentant O, NH et/ou N(CH₃) et m étant égal à 3, 4, 5 ou 6,
- R² représente X(C₂H₄X)ₙ avec X représentant O, NH et/ou N(CH₃) et n étant égal à 3, 4, 5 ou 6, ou
- R¹ et R² représentent X(C₂H₄X)_{p/2}YX(C₂H₄X)_{p/2} avec X représentant O, NH et/ou N(CH₃), p étant égal à 2 ou 4 et Y représentant un groupe cyclohexylène, phénylène, naphtylène, pyridylène ou thiophénylène, et
- R³ représente un atome d'hydrogène ou un groupe alkyle en C₁ à C₃, et
à récupérer ensuite le césium extrait dans cette phase liquide.

7. Procédé selon la revendication 6, caractérisé en ce que l'on récupère ensuite le césium dans une solution aqueuse de réextraction par mise en contact de la phase liquide ayant extrait le césium avec une solution aqueuse.

8. Procédé selon la revendication 7, caractérisé en ce que la solution aqueuse ce réextraction est de l'eau distillée et désionisée.

9. Procédé selon l'une quelconque des revendications 7 et 8, caractérisé en ce que la phase liquide immiscible forme une membrane liquide et en ce que l'on met en contact la solution aqueuse contenant le césium avec une surface de cette membrane et en ce que l'on met en contact la solution aqueuse de réextraction avec la surface opposée de cette membrane liquide.

10. Procédé selon l'une quelconque des revendications 6 à 9, caractérisé en ce que la solution aqueuse de départ est un effluent aqueux contenant du césium, avec ou sans sodium et/ou strontium, provenant d'une installation de retraitement de combustibles irradiés.

11. Procédé pour séparer les actinides et l'américium trivalent présents dans une solution aqueuse, caractérisé en ce qu'il consiste à mettre en contact cette solution aqueuse avec une phase liquide immiscible comprenant un calix[4]arène-bis-couronne selon l'une quelconque des revendications 1 à 4 et à récupérer ensuite les actinides extraits dans la phase liquide immiscible.

## Patentansprüche

1. Bis(krone)calix[4]aren der Formel: 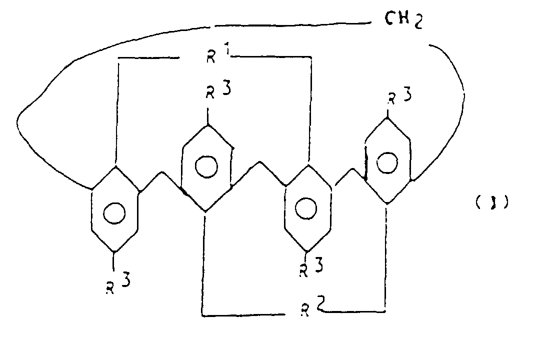 worin
- R¹ für X(C₂H₄X)ₘ steht, wobei X O, NH und/oder N(CH₃) bedeutet und m gleich 3, 4, 5 oder 6 ist,
- R² für X(C₂H₄X)ₙ steht, wobei X O, NH und/oder N(CH₃) bedeutet und n gleich 3, 4, 5 oder 6 ist, oder
- R¹ und R² für X(C₂H₄X)_{p/2}YX(C₂H₄X)_{p/2} stehen, wobei X O, NH und/oder N(CH₃) bedeutet, p gleich 2 oder 4 ist und Y eine Cyclohexylen-, Phenylen-, Naphtylen-, Pyridylen oder Thiophenylengruppe bedeutet, und
- R³ ein Wasserstoffatom oder eine C₁- bis C₃-Alkylgruppe bedeutet.

2. Bis(krone)calix[4]aren nach Anspruch 1, dadurch gekennzeichnet, daß R¹ und R² für O(C₂H₄O)₄ stehen und R³ ein Wasserstoffatom bedeutet.

3. Bis(krone)calix[4]aren nach Anspruch 1, dadurch gekennzeichnet, daß R¹ und R² für O(C₂H₄O)₅ stehen und R³ ein Wasserstoffatom bedeutet.

4. Bis(krone)calix[4]aren nach Anspruch 1, dadurch gekennzeichnet, daß R¹ und R² 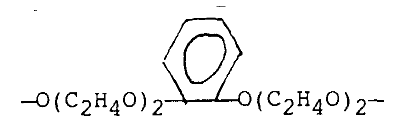 bedeuten und R³ ein Wasserstoffatom bedeutet.

5. Verfahren zur Herstellung eines Bis(krone)calix[4]arens, bei dem X für O steht, nach einem der Ansprüche 1 bis 4, dadurch gekennzeichnet, daß es darin besteht, ein Calix[4]aren der Formel: 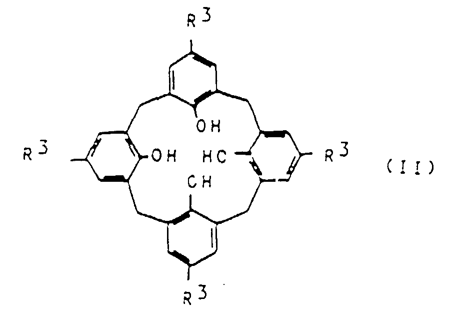 worin R³ die in Anspruch 1 gegebene Bedeutung hat, reagieren zu lassen
- mit einem Tetra-, Penta- oder Hexaethylenglycol der Formel:
HOH₂C-(CH₂OCH₂)ₚ-CH₂OH (III)
worin p = 2, 3, 4 oder 5, oder
- mit zwei verschiedenen Glycolen der Formel (III) oder
- mit einem Glycol der Formel
[HOCH₂(CH₂OCH₂)₂O]₂Y (IV)
worin Y die in Anspruch 1 gegebene Bedeutung hat.

6. Verfahren zur Abtrennung des Caesiums aus einer wäßrigen Lösung, dadurch gekennzeichnet, daß es darin besteht, diese wäßrige Lösung in Kontakt mit einer damit nicht mischbaren flüssigen Phase zu bringen, die ein Bis(krone)calix[4]aren der Formel 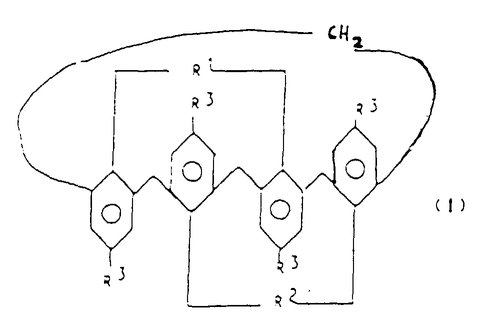 enthält, worin
- R¹ für X(C₂H₄X)ₘ steht, wobei X O, NH und/oder N(CH₃) bedeutet und m gleich 3, 4, 5 oder 6 ist,
- R² für X(C₂H₄X)ₙ steht, wobei X O, NH und/oder N(CH₃) bedeutet und n gleich 3, 4, 5 oder 6 ist, oder
- R¹ und R² für X(C₂H₄X)_{p/2}YX(C₂H₄X)_{p/2} stehen, wobei X O, NH und/oder N(CH₃) bedeutet, p gleich 2 oder 4 ist und Y eine Cyclohexylen-, Phenylen-, Naphtylen-, Pyridylen oder Thiophenylengruppe bedeutet, und
- R³ ein Wasserstoffatom oder eine C₁- bis C₃-Alkylgruppe bedeutet,
enthält, und anschließend das in diese flüssige Phase extrahierte Caesium zurückzugewinnen.

7. Verfahren nach Anspruch 6, dadurch gekennzeichnet, daß man das Caesium anschließend in einer wäßrigen Rückextraktionslösung dadurch zurückgewinnt, daß man die flüssige Phase, die das Caesium extrahiert hat, mit einer wäßrigen Lösung in Kontakt bringt.

8. Verfahren nach Anspruch 7, dadurch gekennzeichnet, daß die wäßrige Rückextraktionslösung destilliertes und deionisiertes Wasser ist.

9. Verfahren nach einem der Ansprüche 7 und 8, dadurch gekennzeichnet, daß die nicht mischbare flüssige Phase eine flüssige Membran bildet, daß man die Caesium enthaltende wäßrige Lösung mit einer Oberfläche dieser Membran in Kontakt bringt und daß man die wäßrige Rückextraktionslösung mit der entgegengesetzten Oberfläche dieser flüssigen Membran in Kontakt bringt.

10. Verfahren nach einem der Ansprüche 6 bis 9, dadurch gekennzeichnet, daß die wäßrige Lösung, von der ausgegangen wird, ein Abwasser ist, das Caesium zusammen mit oder ohne Natrium und/oder Strontium enthält, und das aus einer Anlage zur Wiederaufarbeitung bestrahlter Kernbrennstoffe stammt.

11. Verfahren zur Abtrennung der Actiniden und des dreiwertigen Americiums, die in einer wäßrigen Lösung vorhanden sind, dadurch gekennzeichnet, daß es darin besteht, diese wäßrige Lösung in Kontakt mit einer damit nicht mischbaren flüssigen Phase zu bringen, welche ein Bis(krone)calix[4]aren nach einem der Ansprüche 1 bis 4 enthält, und anschließend die in die nicht mischbare flüssige Phase extrahierten Actiniden zurückzugewinnen.

## Claims

1. Bis-crown calix[4]arene of formula: 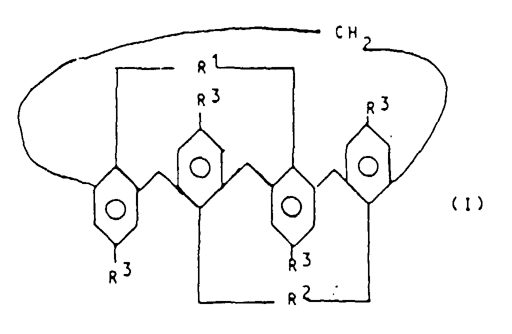 in which
- R¹ represents X(C₂ H₄ X)ₘ with X representing O, NH and/or N(CH₃) and m being equal to 3, 4, 5 or 6,
- R² represents X(C₂ H₄ X)ₙ with X representing 0, NH and/or N(CH₃) and n being equal to 3, 4, 5 or 6, or
- R¹ and R² represent X(C₂ H₄ X)_{p/2} YX(C₂ H₄ X)_{P/2} with X representing 0, NH and/or N(CH₃), p being equal to 2 or 4 and Y representing a cyclohexylene, phenylene, naphthylene, pyridylene or thiophenylene group and
- R³ represents a hydrogen atom or a C₁ to C₃ alkyl group.

2. Bis-crown calix[4]arene according to claim 1, characterized in that R¹ and R² represent O(C₂ H₄ O)₄ and R³ represents a hydrogen atom.

3. Bis-crown calix[4]arene according to claim 1, characterized in that R¹ and R² represent O(C₂ H₄ O)₅ and R³ represents a hydrogen atom.

4. Bis-crown calix[4]arene according to claim 1, characterized in that R¹ and R² represent 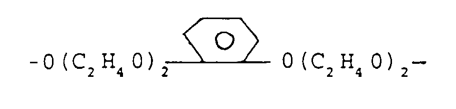 and R³ represents a hydrogen atom.

5. Proces for the preparation of a bis-crown calix[4]arene for which X represents O according to any one of the claims 1 to 4, characterized in that it consists of reacting a calix[4]arene of formula: 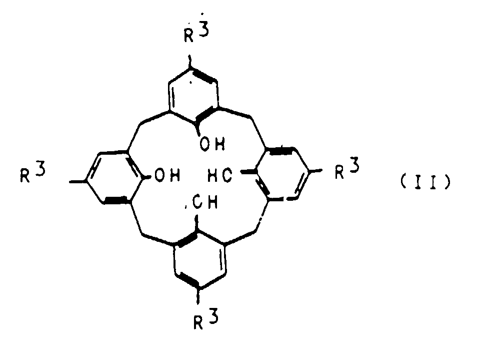 in which R³ has the meaning given in claim 1,
- with a tetra, penta or hexa ethylene glycol of formula:
HOH₂ C- (CH₂ 0CH₂)ₚ -CH₂ OH (III)
in which p=2, 3, 4 or 5, or
- with two different glycols of formula (III), or
- with a glycol of formula
[HOCH₂ (CH₂ 0CH₂]₂ -Y (IV)
in which Y has the meaning given in claim 1.

6. Process for the preparation of cesium from an aqueous solution, characterized in that it consists of contacting this aqueous solution with an immiscible liquid phase comprising a bis-crown calix[4]arene of formula: 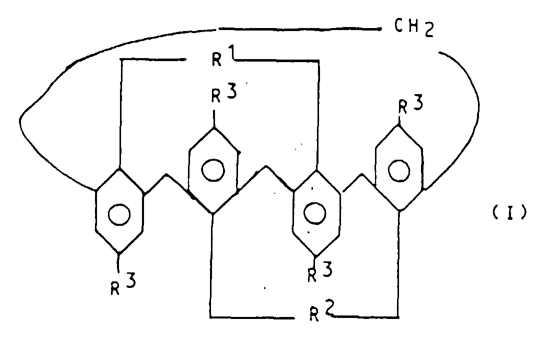 in which
- R¹ represents X(C₂ H₄ X)ₘ with X representing O, NH and/or N(CH₃) and m being equal to 3, 4, 5 or 6,
- R² represents X(C₂ H₄ X)ₙ with X representing 0, NH and/or N(CH₃) and n being equal to 3, 4, 5 or 6, or
- R¹ and R² represent X(C₂ H₄ X)_{p/2} YX(C₂ H₄ X)_{p/2} with X representing 0, NH and/or N(CH₃), p being equal to 2 or 4 and Y representing a cyclohexylene, phenylene, naphthylene, pyridylene or thiophenylene group and
- R³ represents a hydrogen atom or a C₁ to C₃ alkyl group, and
then recovering the cesium extracted in this liquid phase.

7. Process according to claim 6, characterized in that recovery then takes place of the cesium in an aqueous reextraction solution by contacting the liquid phase having extracted the cesium with an aqueous solution.

8. Process according to claim 7, characterized in that the aqueous reextraction solution is distilled, deionized water.

9. Process according to either of the claims 7 and 8, characterized in that the immiscible liquid phase forms a liquid membrane and in that the aqueous solution containing the cesium is contacted with one surface of said membrane and in that the aqueous reextraction solution is contacted with the opposite surface of said liquid membrane.

10. Process according to any one of the claims 6 to 9, characterized in that the aqueous starting solution is an aqueous effluent containing cesium, with or without sodium and/or strontium, obtained from an irradiated fuel reprocessing installation.

11. Process for separating actinides and trivalent americium present in an aqueous solution, characterized in that it consists of contacting said aqueous solution with an immiscible liquid phase comprising a bis-crown calix[4]arene according to any one of the claims 1 to 4 and then recovering the extracted actinides in the immiscible liquid phase.
